# EUROPÄISCHE PATENTSCHRIFT

(11) **EP 1 069 955 B1**
(45) Veröffentlichungstag und Bekanntmachung des Hinweises auf die Patenterteilung: **13.11.2002**
(21) Anmeldenummer: 99917967.4
(22) Anmeldetag: 08.04.1999
(51) Int. Cl.: B03C 5/02

(54) **VERFAHREN UND VORRICHTUNG ZUR MANIPULIERUNG VON MIKROPARTIKELN IN FLUIDSTRÖMUNGEN**
METHOD AND DEVICE FOR MANIPULATING MICROPARTICLES IN FLUID FLOWS
PROCEDE ET DISPOSITIF PERMETTANT DE MANIPULER DES MICROPARTICULES CONTENUES DANS DES ECOULEMENTS FLUIDIQUES

(30) Priorität: 08.04.1998 DE 19815882
(43) Veröffentlichungstag der Anmeldung: 24.01.2001
(73) Patentinhaber: Evotec OAI AG, 22525 Hamburg (DE)
(72) Erfinder: FUHR, Günter, D-13187 Berlin (DE); HAGEDORN, Rolf, D-13057 Berlin (DE); MÜLLER, Torsten, D-12439 Berlin (DE); SCHNELLE, Thomas, D-10243 Berlin (DE); GRADL, Gabriele, D-10557 Berlin (DE)
(74) Vertreter: Hertz, Oliver, Dr.
(86) Internationale Anmeldenummer: EP9902380
(87) Internationale Veröffentlichungsnummer: WO99052640

(56) Entgegenhaltungen:
- DE-A- 4 127 405
- DE-C- 19 605 830
- US-A- 5 626 734

## Beschreibung

Die Erfindung betrifft ein System zur Manipulierung von Mikropartikeln in Fluidstromungen, insbesondere ein Verfahren zur Bewegung von Mikropartikeln wie z. B. von biologischen Zellen zwischen verschiedenen Fluiden beispielsweise für Sortier-, Behandlungs- oder Halterungszwecke und eine mikrosystemtechnische Vorrichtung zur Implementierung des Verfahrens.

Für viele biologische, medizinische, pharmakologische aber auch nicht-biologische Anwendungen ist die präzise Beladung mit Substanzen und berührungslose Halterung mikroskopischer kleiner Teilchen, wie biologische Zellen oder Zellhaufen, Latexpartikeln oder andere Microbeads in freier Flüssigkeit von Bedeutung. Die häufigste Lösung ist das Aufwachsen von Zellen auf einem festen Substrat, das dann mit der geforderten Genauigkeit mit einer Lösung überspült wird bzw. die Halterung in einem Sieb oder an Kapillaroffnungen. Nachteilig an diesem Verfahren ist der mechanische Oberflächenkontakt und die Schwierigkeit, viele Objekte in gleicher Weise und nacheinander zu behandeln. Besondere Schwierigkeiten bereitet es, Mikroobjekte ohne Oberflächenberührung für sehr kurze und einstellbare Zeiten einer anderen Lösung auszusetzen und sie dann in das ursprüngliche Medium rückzuführen. Bisher wird das durch aufwendige Wasch- und Zentrifugierschritte erreicht.

Ebenfalls benutzt werden sogenannte "Laserstrahl-Tweezers", mit denen es gelingt, Partikel in freier Losung an einer mikrometergenauen Position zu halten oder definiert zu verschieben [siene A. Ashkin et al. in "Optics Lett.", Bd. 11, S. 288 (1986)]. Nachteilig ist, daß dieses Prinzip einen betrachtlichen externen Apparateaufwand erfordert, der den Vorteilen der Miniaturisierung von Systemen entgegensteht und kostenintensiv ist. Hinzu kommt die Belastung des Objektes im Fokusbereich.

Eine Alternative stellen elektrische Mikrofeldkäfige dar, in denen Mikropartikeln und Zellen über Polarisationskräfte analog zu den "Laser-Tweezers" gehalten werden können [G. Fuhr et al. in "Naturwiss.", Bd. 81, S. 528 (1994)]. Bei derartigen Systemen befindet sich jedoch nur eine Lösung in dem System, so daß eine Überführung der Mikropartikel in ein anderes Medium nur durch Flüssigkeitsaustausch erfolgen kann, was längere Zeiten bis zur nächsten Benutzung und ggf. gesonderte Reinigungsschritte erfordert. Das Halten eines Partikels in einer Halterungs- oder Parkposition laßt sich zwar mit einer Laser-Pinzette bewerkstelligen, ist jedoch für mehrere Teilchen technisch nicht sinnvoll realisierbar. Zudem befindet sich das Objekt während der Parkzeit unter einer permanenten Strahlenbelastung.

In Mikrosystemen wurden magnetisch geladene Teilchen über rechtwinklig zu den Kanalen wirkende Magnetfelder oder Ultraschallquellen von einer Losung in eine andere überführt [siehe G. Blankenstein in "Scientific and Clinical Applications of Magnetic Carriers", Hrsg. Hafeli et al., Plenum Press New York 1997 (Kap. 16, S. 233 ff.)] . Beide Techniken eigenen sich jedoch nur sehr bedingt zur Miniaturisierung, erlauben keine Fokussierung der Kraftwirkung auf die Teilchen und lassen sich schwer in integrierter Form mit den Techniken der Halbleiterstrukturierungsverfahren umsetzen. Ferner ist diese Technik an eine für biologische Objekte ggf. physiologisch störende Beladung mit magnetischen Teilchen gebunden.

Aus DE-OS 41 43 573 ist eine Vorrichtung zur Trennung von Gemischen mikroskopisch kleiner Teilchen in einer Flüssigkeit bekannt, bei der die Teilchen elektrischen Wanderfeldern ausgesetzt werden, unter deren Wirkung die Teilchen aus einer Strömung der Flüssigkeit ausgekoppelt werden. Diese Vorrichtung besitzt die folgenden Nachteile. Zur Erzeugung der Wanderwellen ist eine Vielzahl von Mikroelektroden erforderlich, so daß sich mit den jeweiligen separaten Ansteuerungen ein komplexer Aufbau ergibt. Die Mikroelektroden sind in einem Bereich angeordnet, der wesentlich größer als die auszukoppelnden Teilchen ist. Die Wanderwellen verursachen in der Flüssigkeit Temperaturgradienten, durch die störende Querströmungen entstehen. Durch diese Querströmungen und ggf. vorhandene weitere Strömungensinhomogenitäten bewegen sich die Teilchen nicht auf definierten Bahnen. Zur Kompensation dieser örtlich undefinierten Auskopplung muß diese sich über einen relativ weiten Bereich in Strömungsrichtung erstrecken. Dadurch wiederum werden ganze Teilchengruppen ausgekoppelt, oder die Teilchen müssen sich mit großen Abständen durch das Mikrosystem bewegen, so daß die Verarbeitung großer Teilchenzahlen verzögert wird.

Mit den bekannten Techniken ist es somit bisher nicht oder nur beschränkt möglich, Mikroteilchen von einer Flüssigkeit in eine oder mehrere andere und zurück zu überführen oder eine berührungsfreie Zwischenlagerung in einem Mikrosystem vorzunehmen.

US-A-5 626 734 offenbart ein Verfahren und eine Vorrichtung zum Trennen von Mikropartikeln gemäß ersten Teil der Ansprüche 1 und 8.

Aufgabe der Erfindung ist es daher, ein verbessertes Verfahren zur Manipulierung von Mikropartikeln in Fluidströmungen anzugeben, das einen erweiterten Einsatzbereich besitzt und insbesondere mit hoher Geschwindigkeit seriell und parallel einsetzbar ist sowie elektrisch steuerbare Verfahren zur berührungsfreien Halterung und zur Überführung von Mikropartikeln in verschiedene Medien ermöglicht. Aufgabe der Erfindung ist es auch, eine Vorrichtung zur Durchführung des Verfahrens bereitzustellen, die einen vereinfachten Aufbau und eine vereinfachte und zuverlässige Ansteuerung besitzt, und zur Ausbildung definierter Bewegungsbahnen der zu manipulierenden Mikropartikel eingerichtet ist.

Diese Aufgabe wird durch ein Verfahren mit den Merkmalen gemäß Patentanspruch 1 und eine Vorrichtung mit den Merkmalen gemäß Patentanspruch 8 gelöst. Vorteilhafte Ausführungsformen der Erfindung ergeben sich aus den abhängigen Ansprüchen.

Der Erfindung liegt die Idee zugrunde, Mikropartikel in einem strömenden Fluid elektrischen Feldkräften auszusetzen. Die elektrischen Feldkräfte werden durch mindestens eine elektrische Feldbarriere ausgeübt, gegen die die Mikropartikel mit dem strömenden Fluid bewegt werden und die eine Bewegungsänderung der Mikropartikel mit einer von der Strömungsrichtung abweichenden Richtung bewirkt. Die elektrische Feldbarriere wird z.B. mit mindestens einem Paar bandförmiger Mikroelektroden erzeugt, das an gegenüberliegenden Begrenzungen des strömenden Fluids angeordnet ist und mit einer hochfrequenten Wechselspannung beaufschlagt wird. Die Amplitude der Wechselspannung bzw. die Feldbarriere ist so hoch gewählt, daß Mikropartikel, die abgelenkt werden sollen, nicht zwischen die Elektroden gelangen können. Das Fluid mit den darin suspendierten Mikropartikeln strömt durch einen Kanal mit mindestens einer seitlichen Öffnung, zu der mindestens ein Mikropartikel entlang der elektrischen Feldbarriere bewegt wird. An die Öffnung grenzt ein weiterer Kanal mit einem stromenden Fluid oder ein schleifenförmiger Abzwelg (sog. Parkschleife) des ersten Kanals. An der Öffnung berühren sich die Fluidströmungen der jeweiligen Kanäle. Bei Realisierung des Fluidströmungssystems mit laminaren Strömungen findet jedoch keine Durchmischung der Fluide statt. Die laminaren Strömungen werden vorzugsweise in Mikrosystemen oder mit kapillarförmigen Kanälen realisiert. Ein besonderer Vorteil der Erfindung besteht darin, daß die strömenden Fluide an den Öffnungen zwischen den Kanälen Grenzflächen ausbilden, die von den zu manipulierenden Mikropartikeln durchlaufen werden können.

Die elektrischen Feldkräfte werden allgemein durch 3-dimensional angeordnete Elektrodeneinrichtungen über oder an den Öffnungen zwischen den Kanälen zur Überführung der Objekte in einen oder mehrere Nachbarkanäle oder Parkschleifen durch An-legen von Hochfrequenzspannungen bei permanenter hydrodynamischer Durchströmung des Systems ausgeübt. Die Ansteuerung der als Ablenksysteme funktionierenden Elektrodeneinrichtungen kann computerbasiert erfolgen und erlaubt minimale Manipulationszeiten im ms-Bereich. Die Bewegung kann in freier Lösung ausgeführt werden, ohne eine mechanische Berührung oder Führung des Objektes. Das Verfahren arbeitet ohne Interferenz mit den üblichen optischen Meßmethoden und vermeidet daher Schäden an lebenden biologischen Objekte, wie z. B. Zellen. Die Aufenthaltsdauer der Teilchen in den Kompartimenten oder Kanalabschnitten läßt sich extern festigen. Typische Bahndurchmesser bzw. Auslenkungen liegen im Bereich von 50 nm und einigen 100 µm oder mehr. Es ist keine Feedback-Kontrolle oder Beobachtung der Objekte erforderlich (sie kann jedoch zusätzlich erfolgen.)

Besondere Vorteile der Erfindung bestehen darin, daß mit einem relativ einfachen Elektrodenaufbau (im einfachsten Fall: mit einem Paar von Elektrodenstreifen) eine zielgenaue, zuverlässige und schnelle Partikelmanipulierung erzielt wird. Störende Querströmungen werden vermieden. Die Elektroden können mit einer ausreichend hohen Wechselspannung beaufschlagt werden, so daß die Partikel sicher auf der stromaufwärts gelegenen Seite der Feldbarriere bleiben und zur seitlichen Öffnung geführt werden. Die Elektroden besitzen charakteristische Dimensionen, die kleiner oder gleich der Dimensionen der zu manipulierenden Partikel sind. Die erfindungsgemäße Partikelmanipulierung erlaubt eine Bewegung der Partikel in die und aus der Strömung, also auch eine Partikelrückführung aus einer benachbarten Strömung.

Weitere Vorteile und Einzelheiten der Erfindung werden im folgenden unter Bezug auf die beigefügten Zeichnungen beschrieben. Es zeigen:
- Fig. 1:: eine Perspektivansicht einer ersten Ausführungsform eines erfindungsgemäßen Fluidströmungssystems;
- Fiq. 2:: eine Draufsicht auf ein Fluidströmungssystem gemäß Fig. 1;
- Fign. 3 bis 5:: Draufsichten auf Fluidströmungssysteme gemäß einer zweiten, dritten und vierten Ausführungsform der Erfindung;
- Fig. 6:: eine Draufsicht auf ein Fluidströmungssystem gemäß einer Ausführungsform mit einem schleifenförmigen Abzweig; und
- Fign. 7 und 8:: Draufsichten auf Fluidströmungssysteme gemäß einer Ausführungsform der Erfindung mit schleifenförmigen Strömungen zwischen zwei Kanälen.

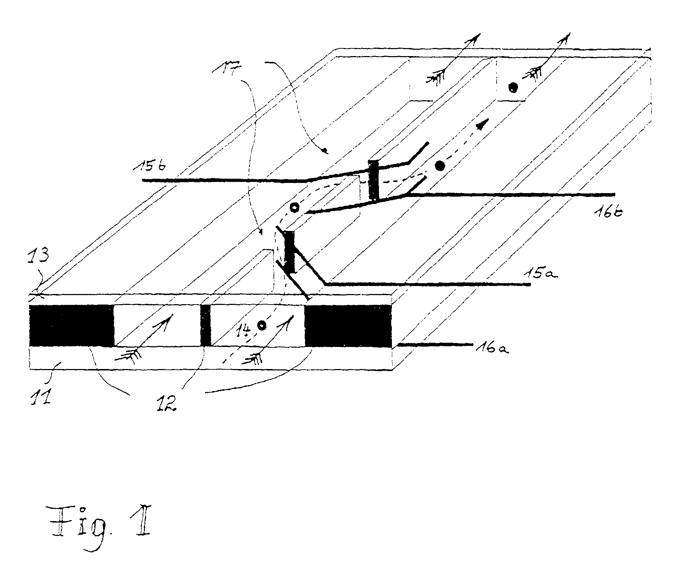

Bei den illustrierten Beispielen handelt es sich stets um dreidimensionale Anordnungen von Mikroelektroden, mit denen barriereartige elektrische Hochfrequenzfelder in Kanälen erzeugt werden. In der perspektivischen Darstellung ist ein derartiges System stellvertretend in Figur 1 dargestellt. Die Beispiele zeigen lediglich 1-, 2- oder 3-Kanalsysteme, Die Erfindung ist jedoch durch beliebige weitere Kombinationen erweiterbar. Die Erfindung wird am Beispiel strömender Flüssigkeiten erlautert, ist jedoch bei genügend starken Feldkräften auch mit anderen Fluiden realisierbar. Die Erfindung ist nicht auf die dargestellten ebenen Kanalwänden beschränkt, sondern auch mit Kanälen anderer, z. B. runder, Querschnitte realisierbar.

Figur 1 zeigt eine perspektivische Ansicht (Ausschnitt) eines 2-Kanalsystems bestehend aus einem Bodensubstrat 11, auf dem in planarer Weise die Mikroelektroden oder Elektrodenabschnitte 16a, 16b (mit geraden Zuleitungen gezeigt) angeordnet sind, dem die Kanalwände bildenden Spacer 12 und einem Decksubstrat 13 (transparent dargestellt, transparent oder nichttransparent realisierbar), auf dessen zum Kanal weisenden Seite ebenfalls planar Mikroelektroden oder Elektrodenabschnitte 15a, 15b (mir geraden Zuleitungen gezeigt) angeordnet sind.

Der Spacer 12 bildet einen rechten (ersten) und einen linken (zweiten) Kanal. Die mittlere Trennwand besitzt Öffnungen 17. Jeder Öffnung 17 ist eine Elektrodeneinrichtung bestehend aus den jeweiligen Elektrodenabschnitten 15a, 16a bzw. 15b, 16b zugeordnet. Die Elektrodenabschnitte erstrecken sich jeweils in einem Kanalabschnitt stromaufwärts von der jeweiligen Öffnung von einer der Öffnung gegenüberliegenden Wand bis zur Öffnung bzw. vorzugsweise durch diese hindurch bis in den benachbarten Kanal. Damit definieren die Elektrodenabschnitte eine Bezugsebene, die senkrecht auf der Fläche des Bodensubstrats 11 und unter einem Winkel zur Kanallängsrichtung steht.

Zwischen den Elektrodenabschnitten 15a, 16a und 15b, 16b wird eine Wechselspannung (Frequenz: kHz bis MHz, Amplitude: 0.1 bis 50 v) angelegt. Die Frequenz wird in Abhängigkeit von den dielektrischen Eigenschaften der Mikropartikel oder Teilchen derart ausgewählt, daß diese eine negative Polarisation, d.h. negative Dielektrophorese, aufweisen, und vom Hochfrequenzfeld abgestoßen werden. Alternativ ist es auch möglich, die Frequenz so zu wählen, daß positive Dielektrophorese stattfindet (Anziehung), wobei dann die zu einer Öffnung gehörenden Elektrodenabschnitte stromaufwärts im jeweils anderen Kanal anzuordnen wären. Die negative Dielektrophorese besitzt jedoch entscheidende Vorteil bei der berührungsfreien Manipulierung der Mikropartikel.

Im Bereich der genannten Bezugsebene wird somit ein abstoßendes Feld als Barriere gebildet, das aufgrund der Neigung gegenüber der Kanallängsrichtung im Zusammenwirken mit der Strömung eine Kraftwirkung auf die Teilchen hin zur Öffnung 17 verursacht.

Im vorliegenden Beispiel werden die Kanäle in gleicher Richtung (Pfeile) von verschiedenen Flüssigkeiten durchströmt. Über den ersten Kanal werden suspendierte Teilchen (z.B. auch lebende Zellen) mit einer Trägerflüssigkeit eingespült. Im zweiten Kanal strömt eine Behandlungsflüssigkeit (z. B. ein Beladungsmedium mit einer gelösten Substanz, mit der die Teilchen beladen werden sollen).

Ein Teilchen 14 bewegt sich auf der gestrichelt gezeichneten Bahn. Zur definierten Behandlung der Mikropartikel werden dese durch die erste Öffnung 17 in den zweiten Kanal bewegt. Über die Strömungsgeschwindigkeit und die Anordnung der ablenkenden Elektrodenabschnitte 15a, 16a bzw. 15b , 16b lassen sich die Partikel für eine definierte Zeit in das Beladungsmedium überführen. In der Regel erfolgt dieser Vorgang bei Strömungsgeschwindigkeiten von einigen bis zu einigen hundert µm/s. Die Verweildauer im Beladungsmedium liegt damit in Abhängigkeit vom Abstand der Ablenkelektroden im ms bis s-Bereich.

Die Rückführung vom zweiten Kanal in den ersten Kanal erfolgt analog an der zweiten Öffnung 17.

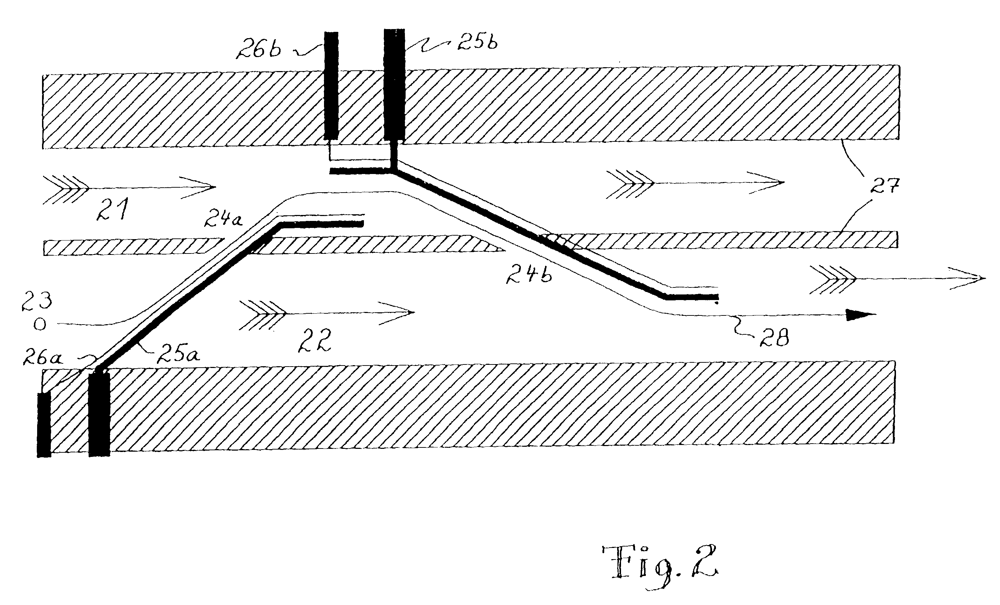

Figur 2 zeigt eine Draufsicht auf das in Figur 1 beschriebene System. Die beiden Kanäle 21, 22 werden von links nach rechts durchströmt. Die Kanalwände bildet ein Spacer 27. Die Partikeln 23 werden bei angeschaltetem Feld der Bahn 28 erfolgen. Anderenfalls wechseln sie nicht in den Nachbarkanal über. Die Elektrodenabschnitte 25a, 26a und 25b, 26b (auch Ablenkelektrodenpaare genannt) sind hier schematisch dargestellt, d.h., die dünne Linie stellt die untere Elektrodenebene 26a, 26b dar und die dickere Linie die obere Elektrodenebene 25a, 25b. Die Breite der Elektroden kann im Bereich zwischen einigen 100 nm bis zu etwa 100 µm liegen (typischerweise 10 bis 20 µm). Die Größe der Partikeln 23 (nm bis mm) bestimmt die Höhe der Kanäle. Günstige Werte sind etwa das 2- bis 20-fache des Partikeldurchmessers. Zur Minimierung von elektrischen Verlusten sind die Zuführung zu den Ablenkelektroden nicht untereinander, sondern möglichst weit seitlich versetzt anzuordnen. Wird die Ablenkeinheit 25b, 26b abgeschaltet, so verbleiben die Partikeln in der Lösung des Kanals 21. Über den Abstand der Öffnungen 24a, 24b oder die Strömungsgeschwindigkeit kann die Verweilzeit in Kanal 21 festgelegt werden.

Die Kanäle besitzen Dimensionen, die in Abhängigkeit von der Fluidviskosität (Bereitstellung laminarer Strömungen) ausgewählt sein konnen. Bevorzugte charakteristische Dimensionen liegen im Bereich von Sub-µm bis mm, vorzugsweise einige µm bis 0.5 mm, z. B. 200 µm.

Die Elektrodenabschnitte sind bandförmig dargestellt, können aber auch jede andere Form haben, die die Kraftwirkung hin zu den Öffnungen in der Kanalwand sicherstellt.

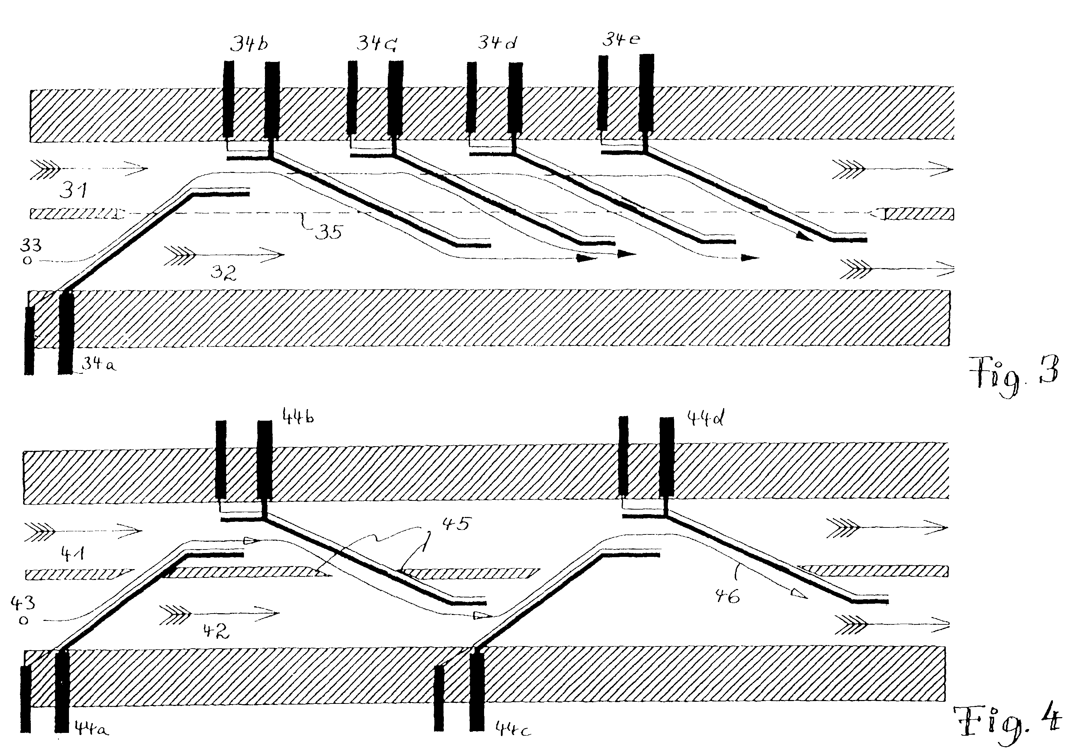

Figur 3 zeigt einen besonderen Vorteil der Erfindung. In Mikrokanälen mit einem Durchmesser <1/2 mm findet namlich keine gegenseitige Störung der Flüssigkeitsströmungen statt (keine Vermischung). Strömungen bleiben über große Strecken laminar. Im dargestellten Beispiel wird dieser Effekt genutzt, um die Partikeln aus Figur 1 und 2 temporär in eine andere Lösung zu überführen. Die Trennwand zwischen den Kanälen 31 und 32 bildet hier eine mehrere µm oder auch einige hundert µm lange Öffnung 35. Bei einer Durchströmung des Kanals in gleicher Richtung kommt es an dieser Berührungsfläche aus o.g. Gründen nicht zu einer Vermischung. Über die Ablenkeinheit 34a läßt sich ein Partikel 33 vom Kanal 32 in den Kanal 31 überführen. Über die Ablenkeinheiten 34b-e kann die Dauer der Verweilzeit im Medium des Kanals 31 bestimmt werden. Die Teilchen bewegen sich auf den mit Pfeilen eingezeichneten Trajektorien.

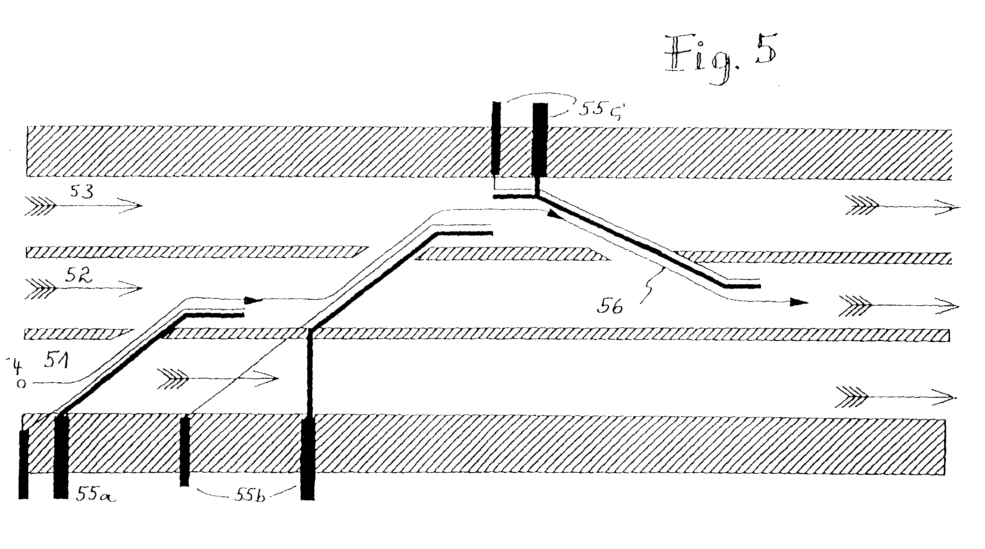

Figur 4 zeigt eine Anordnung, bei der die Überführung eines Partikels 43 von Kanal 42 in Kanal 41 und zurück mehrfach erfolgen kann. Das System kann man auch weiter forgesetzt realisiert werden. Die Teilchen folgen der Bewegungsbahn 46. Im ersten Element 44a, 44b befindet sich eine Trennwand 45. Die zweite Ablenkvorrichtung 44c, 44c kommt ohne dieses Element aus. Je nach Abstand der Ablenksysteme kann auch beim ersten Übergangsbereich auf das Trennwandelement verzichtet werden.

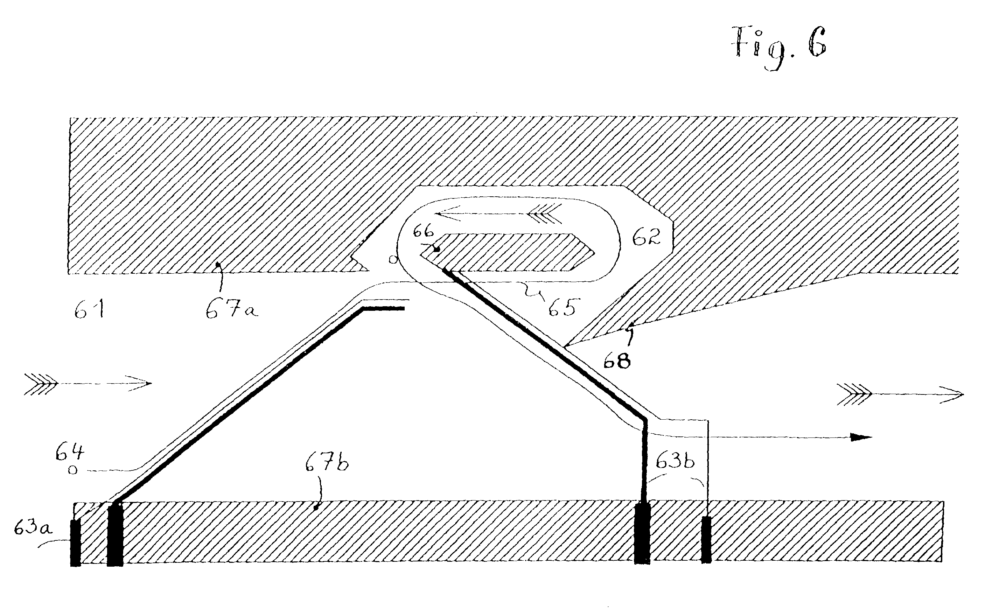

Für biochemische und zellbiologisch-medizinische Aufgabenstellungen ist es häufig von Bedeutung, Objekte definiert und steuerbar kurzzeitig in mehrere Flüssigkeiten zu überführen. Beispielhaft ist in Fig. 5 ein 3-Kanalsystem dargestellt. Alle Kanäle 51, 52, 53 werden von links nach rechts durchströmt. Die Partikel 54 können über das Ablenksystem 55a in den Kanal 52 und über 55b in den Kanal 53 überführt werden. Über die Ablenk-einheit 55c läßt sich das Teilchen wieder in Kanal 52 zurückführen. Es folgt der Bewegungsbahn 56. Durch Anordnung einer weiteren Ablenkeinheit und Öffnung zwischen den Kanälen 51 und 52 läßt sich das Teilchen auch wieder in den Kanal 51 zurückführen. Nach dem dargestellten Muster können sich weitaus höhere Kanalzahlen und Überführungselemente realisieren.

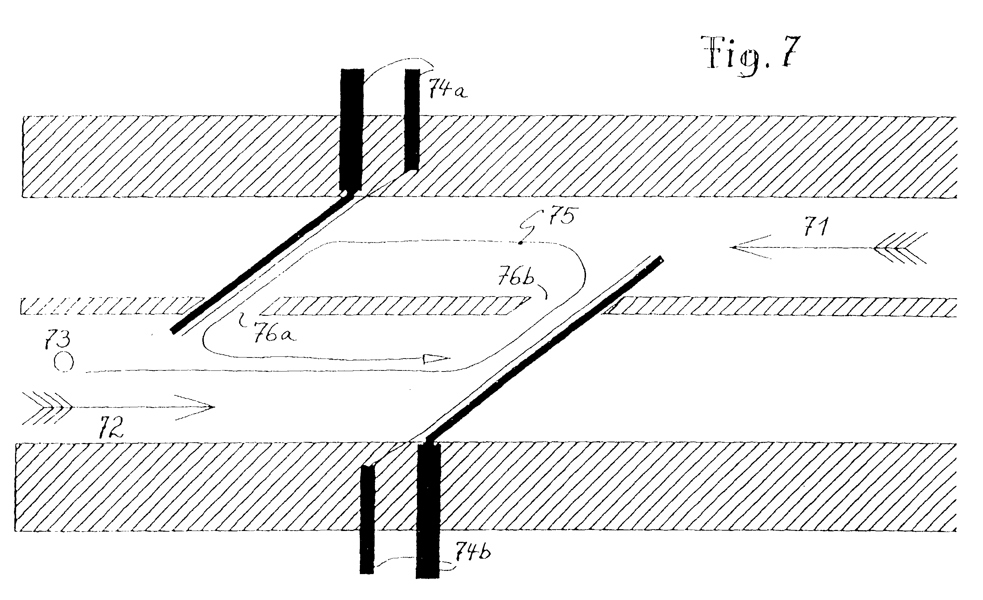

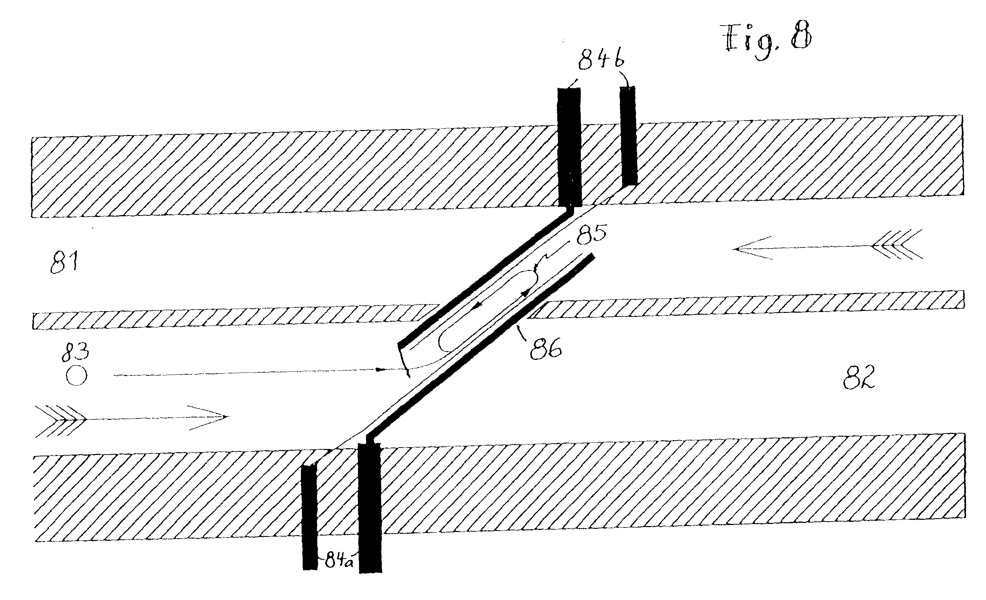

Ein bisher ungelöstes Problem in Mikrofluidiksystemen mit zellbiologischer Anbindung stellen die kurzen Durchflußzeiten dar. Wird beispielsweise ein Partikel vermessen, so mußte bisher entweder die Strömung angehalten werden oder weitere im Kanalsystem befindliche Teilchen werden unwiederbringlich ausgespült. Wird die Strömung angehalten, besteht die Gefahr eines Oberflächenkontaktes und nachfolgende Adhäsion. Aus diesem Grunde ist es wünschenswert, bei permanenter Strömung Parkschleifen für Partikel zu realisieren. In Figur 6 ist ein derartiges Grundelement dargestellt. Der Kanal 61 wird von links nach rechts durchströmt. In einer der Wände (67a) befindet sich ein Ringkanal 62, der durch einen Spacerabschnitt 66 gebildet wird. Der Spacer ragt an der hinteren Seite etwas in der Kanal 68 hinein, so daß ein Teil der Flüssigkeit im Kanal 62 zu zirkulieren beginnt. Ein Partikel 64 kann über die Ablenkelektrode 63a in diese Strömung hineingeführt werden. Falls die Ablenk-elektrode 63b nicht angesteuert wird, verbleibt es in der Ringströmung und bewegt sich auf einer schleifenförmigen Parkbahn 65. Soll das Teilchen entnommen werden, so wird das Ablenksystem 63b angeschaltet und das Partikel verläßt die Parkschleife.

Eine Kombination aus Partikelparkschleife und definierter Überführung in eine andere Lösung erfolgt, wenn zwei Ablenksysteme 74a, 74b über Öffnungen in der gemeinsamen Kanalwand in den jeweils benachbarten kanal 71, 72 hineinragen. Ein Teilchen 73 würde sich bei entgegengesetzter Durchströmung der Kanäle 71, 72 in eine kreisförmige Bewegungsbahn 75 begeben, in der gleichzeitig auch mehrere Partikeln Platz fänden. Durch Abschalten der HF-Spannung an einer oder beiden Ablenksystemen kann das Partikel in den einen oder anderen Lösungsstrom entlassen werden. Diese Anordnung besitzt gleichzeitig den Vorteil, daß in beiden Kanälen unterschiedlich zusammengesetzte Flüssigkeiten benutzt werden können. Über die Zahl der Umläufe des Teilchens läßt sich die Zeit, die es der jeweiligen Substanz ausgesetzt sein soll, einstellen und meßbar reproduzieren. Über zusätzliche Detektionsmaßnahmen an einer oder mehreren Stellen kann die Umlaufzeit und die Zahl der gefangenen Partikeln bestimmt werden. Da kann optisch, aber auch über die Art eines "Coulter Counters" an den Öffnungen 76a, 76b erfolgen. Das System kann man sich auch erweitert, bestehend aus einer Vielzahl solcher Elemente in Serie als auch parallel vorstellen. Es ist damit geeignet, eine Vielzahl von Partikeln zu halten, ihren Aufenthaltsort zu erfassen und sie in vergleichbarer Weise zu behandeln.

Von besonderem Interesse sind sehr kurze Aufenthaltszeiten bzw. Parkschleifen, die in großer Zahl und jeweils nur von einem oder wenigen Partikeln gleichzeitig belegt wären können. Dazu sind die Ablenksysteme 84a, 84b möglichst nahe zueinander und in einer Öffnung 86 zwischen den Kanälen 81, 82 zu plazieren. Werden nun beide Kanäle in entgegengesetzter Richtung durchströmt, so wird das Teilchen 83 der Trajektorie 85 folgen. Der minimale Durchmesser der Bewegungsbahn liegt bei etwa dem Doppelten des Partikeldurchmessers. Geht man davon aus, daß auch Submikrometerteilchen wie Viren auf diese Weise gefangen und von einer Lösung in eine andere periodisch überführt werden können, so liegen die kürzesten Zeiten für einen Umlauf bei einigen ms.

## Patentansprüche

1. Verfahren zur Manipulierung von Mikropartikeln (14, 23, 33, 43, 54, 64, 73, 83) in einem Fluid, das als Strömung mindestens einen ersten Kanal mit mindestens einer seitlichen Öffnung (17, 24a, 24b, 35, 76a, 76b, 86) und mindestens einer Elektrodeneinrichtung (15a, 15b, 16a, 16b, 25a, 26a, 25b, 26b, 34a-e, 44a-d, 55a-c, 63a-b, 74a-b, 84a-b) durchläuft, die zur Erzeugung mindestens einer elektrischen Feldbarriere ausgebildet ist, wobei die Elektrodeneinrichtung (15a, 15b, 16a, 16b, 25a, 26a, 25b, 26b, 34a-e, 44a-d, 55a-c, 63a-b, 74a-b, 84a-b) durch Beaufschlagung mit einer elektrischen Hochfrequenzspannung zumindest zeitweilig so angesteuert wird, daß eine die Strömung durchsetzende elektrische Feldbarriere gebildet wird, unter deren Wirkung ein oder mehrere Mikropartikel (14, 23, 33, 43, 54, 64, 73, 83) am Vorbeitritt an der Elektrodeneinrichtung gehindert werden, **dadurch gekennzeichnet, daß**
die Mikropartikel (14, 23, 33, 43, 54, 64, 73, 83) auf der stromaufwärts gelegenen Seite der Elektrodeneinrichtung (15a, 15b, 16a, 16b, 25a, 26a, 25b, 26b, 34a-e, 44a-d, 55a-c, 63a-b, 74a-b, 84a-b) eine Bewegungsänderung mit einer von der Strömungsrichtung abweichenden Richtung erfahren und gerichtet an den Rand der Strömung zu der seitlichen Öffnung (17, 24a, 24b, 35, 76a, 76b, 86) bewegt werden, wobei jede seitliche Öffnung (17, 24a, 24b, 35, 76a, 76b, 86) einen Übergangsbereich bildet, in dem die Mikropartikel (14, 23, 33, 43, 54, 64, 73, 83) vom jeweils ersten Kanal (22, 32, 42, 51, 61, 72, 82) in einen angrenzenden zweiten Kanal (21, 31, 41, 52, 62, 71, 81) oder mehrere angrenzende zweite Kanäle (52, 53) oder einen Abzweig vom ersten Kanal mit einer schleifenförmigen Strömungsausbildung, oder umgekehrt bewegt werden.

2. Verfahren gemäß Anspruch 1, bei dem jeder Mikropartikel unter der Wirkung der elektrischen Hochfrequenzspannung zwischen mindestens zwei Mikroelektroden (15a, 15b, 16a, 16b, 25a, 26a, 25b, 26b, 34a-e, 44a-d, 55a-c, 63a-b, 74a-b, 84a-b), die die elektrische Feldbarriere erzeugen, und der Strömungskräfte im Fluid in den Übergangsbereich und unter der Wirkung der Strömung jeweils entsprechend im angrenzenden Kanal oder Abzweig in diesem weiterbewegt wird.

3. Verfahren gemäß Anspruch 2, bei dem hochfrequente elektrische Felder örtlich begrenzt in Kanalabschnitten erzeugt werden, die jeweils an einen Übergangsbereich angrenzen oder diesen überlappen.

4. Verfahren gemäß einem der Ansprüche 1 bis 3, bei dem die Strömungen in den Kanälen oder Abzweigen laminare Strömungen sind.

5. Verfahren gemäß einem der Ansprüche 1 bis 4, bei dem die Mikropartikel (54) von einem ersten Kanal (51) in eine Vielzahl zweiter Kanäle (52, 53) partikelspezifisch sortiert werden, indem elektrische Feldbarrieren für jeden Mikropartikel entsprechend einem vorbestimmten Zeitmuster spezifisch so lokal ausgebildet werden, daß eine Bewegung in einen vorbestimmten Kanal der Gruppe der zweiten Kanäle erfolgt.

6. Verfahren gemäß einem der Ansprüche 1 bis 4, bei dem das Fluid im ersten Kanal (22) eine inerte Trägerflüssigkeit und das Fluid im zweiten Kanal (21) eine Behandlungsflüssigkeit ist und die elektrischen Feldbarrieren in vorbestimmter Weise so ausgebildet werden, daß die Mikropartikel (23) entsprechend vorbestimmter Zeitmuster von der Trägerflüssigkeit in die Behandlungsflüssigkeit bewegt, dort behandelt und wieder zurück in die Trägerflüssigkeit bewegt werden.

7. Verfahren gemäß einem der Ansprüche 1 bis 4, bei dem die Mikropartikel (64) entsprechend vorbestimmter Zeitmuster vom ersten Kanal (61) in einen schleifenförmigen Abzweig oder ein Reservoir (62) mit schleifenförmiger Strömung bewegt, dort geparkt und/oder vermessen und wieder zurück in den ersten Kanal bewegt werden.

8. Fluidströmungssystem zur Manipulierung von Mikropartikeln (14, 23, 33, 43, 54, 64, 73, 83) in strömenden Fluiden mit mindestens einem ersten Kanal (22, 32, 42, 51, 61, 72, 82) zur Aufnahme einer Strömung eines Fluids, der mindestens eine seitliche Öffnung (17, 24a, 24b, 35, 76a, 76b, 86) und mindestens eine Elektrodeneinrichtung (15a, 15b, 16a, 16b, 25a, 26a, 25b, 26b, 34a-e, 44a-d, 55a-c, 63a-b, 74a-b, 84a-b) aufweist, die zur Erzeugung mindestens einer die Strömung durchsetzenden elektrischen Feldbarriere ausgebildet ist, **dadurch gekennzeichnet, daß**
die Elektrodeneinrichtung derart gestaltet und mit einer elektrischen Hochfrequenzspannung beaufschlagbar ist, daß für mindestens ein Mikropartikel die elektrische Feldbarriere auf der stromaufwärts gelegenen Seite der Elektrodeneinrichtung eine Führung für eine gerichtete Bewegung des Mikropartikels hin zur seitlichen Öffnung bildet und mindestens ein zweiter Kanal (21, 31, 41, 52, 62, 71, 81) zur Aufnahme einer weiteren Fluidströmung vorgesehen ist, der mindestens eine seitliche Öffnung aufweist, die mit der seitlichen Öffnung des ersten Kanals einen Übergangsbereich bildet.

9. Fluidströmungssystem gemäß Anspruch 8, bei dem im zweiten Kanal mindestens eine weitere Elektrodeneinrichtung (15b, 16b, 25b, 26b, 34b-e, 44b, d, 55b, c, 74a, 84b) vorgesehen ist, die zur Ausbildung mindestens einer elektrischen Feldbarriere quer oder schräg zur Strömungsrichtung im ersten Kanal zur Bewegung mindestens eines Mikropartikels hin zu einer weiteren seitlichen Öffnung eingerichtet ist.

10. Fluidströmungssystem gemäß einem der Ansprüche 8 oder 9, bei dem die ersten und zweiten Kanäle gerade Kapillarkanäle sind, die durch eine Trennwand (12, 27, 45) getrennt sind, in der die seitliche(n) Öffnung(en) ausgebildet sind.

11. Fluidströmungssystem gemäß Anspruch 10, bei dem jede Öffnung mindestens zwei Übergangsbereiche mit jeweils einer Elektrodeneinrichtung bildet.

12. Fluidströmungssystem gemäß einem der Ansprüche 8 bis 11, bei dem die Elektrodeneinrichtung Elektrodenabschnitte umfaßt, die an der Kanalwandung angebracht sind und sich von einer der jeweiligen seitlichen Öffnung gegenüberliegenden Kanalseite stromaufwärts hin zu der jeweiligen seitlichen Öffnung erstrecken.

13. Fluidströmungssystem gemäß Anspruch 12, bei dem die Elektrodenabschnitte bandförmig sind und in den jeweils anderen Kanal ragen.

14. Fluidströmungssystem gemäß einem der Ansprüche 12 oder 13, bei dem einem Übergangsbereich jeweils zwei parallele Elektrodenabschnitte an gegenüberliegenden Bereichen der Kanalwandung zugeordnet sind.

15. Fluidströmungssystem gemäß einem der Ansprüche 8 oder 14, bei dem der zweite Kanal (62) ein schleifenförmiger Abzweig vom ersten Kanal ist.

16. Fluidströmungssystem gemäß einem der Ansprüche 8 oder 14, bei dem eine Vielzahl erster Kanäle und/oder zweiter Kanäle und/oder Abzweige vorgesehen sind, die über Übergangsbereiche verbunden sind, die jeweils eine Elektrodeneinrichtung aufweisen.

17. Verwendung eines Fluidströmungssystems gemäß einem der Ansprüche 8 oder 16, als
- Sortier- oder Trennvorrichtung,
- Behandlungsvorrichtung, und/oder
- Aufbewahrungs- und Halterungsvorrichtung.

## Claims

1. A method of manipulating micro-particles (14, 23, 33, 43, 54, 64, 73, 83) in a fluid, which passes as a flow through at least one first channel with at least one side opening (17, 24a, 24b, 35, 76a, 76b, 86) and at least one electrode arrangement (15a, 15b, 16a, 16b, 25a, 26a, 25b, 26b, 34a-e, 44a-d, 55a-c, 53a-b, 74a-b, 84a-b), which is arranged to generate at least one electric field barrier, wherein the electrode arrangement (15a, 15b, 16a, 16b, 25a, 26a, 25b, 26b, 34a-e, 44a-d, 55a-c, 53a-b, 74a-b, 84a-b) is so controlled by application of an electric high frequency voltage at least intermittently that an electric field barrier extending through the flow is formed, under the action of which one or more micro-particles (14, 23, 33, 43, 54, 64, 73, 83) are prevented from passing by at the electrode arrangement, **characterized in that** the micro-particles (14, 23, 33, 43, 54, 64, 73, 83) experience a change of movement on the upstream side of the electrode arrangement (15a, 15b, 16a, 16b, 25a, 26a, 25b, 26b, 34a-e, 44a-d, 55a-c, 53a-b, 74a-b, 84a-b), with a direction departing from the flow direction and moved in the direction of the edge of the flow to the side opening (17, 24a, 24b, 35, 76a, 76b, 86), wherein each side opening (17, 24a, 24b, 35, 76a, 76b, 86) forms a transition region in which the micro-particles (14, 23, 33, 43, 54, 64, 73, 83) are moved from the respective first channel (22, 32, 42, 51, 61, 72, 82) into an adjoining second channel (21, 31, 41, 52, 62, 71, 81) or a plurality of adjoining second channels (52, 53) or a branch off the first channel with a loop formed flow formation, or vice versa.

2. A method according to claim 1, in which each micro-particle is moved on under the action of the electric high frequency voltage between at least two micro-electrodes (15a, 15b, 16a, 16b, 25a, 26a, 25b, 26b, 34a-e, 44a-d, 55a-c, 53a-b, 74a-b, 84a-b), which generate the electric field barrier, and the flow forces in the fluid in the transition region and under the action of the flow, in each case correspondingly into the adjoining channel or branch.

3. A method according to claim 2, in which high frequency electric fields are generated locally bounded in channel sections which each adjoin a transition region or overlap this.

4. A method according to any of claims 1 to 3, in which the flows in the channels or branches are laminar flows.

5. A method according to any of claims 1 to 4, in which the micro-particles (54) are sorted from a first channel (51) into a plurality of second channels (52, 53) for specific particles, in that electric field barriers for each micro-particle are so formed locally in accordance with a predetermined time pattern that movement takes place into a predetermined channel of the group of second channels.

6. A method according to any of claims 1 to 4, in which the fluid in the first channel (22) is an inert carrier fluid and the fluid in the second channel (21) is a treatment fluid, and the electric field barriers are so formed in a predetermined manner that the micro-particles (23) are moved in accordance with a predetermined time pattern from the carrier fluid into the treatment fluid, are treated there and are moved back into the carrier fluid.

7. A method according to any of claims 1 to 4, in which the micro-particles (64) are moved in accordance with a predetermined time pattern from the first channel (61) into a loop formed branch or a reservoir (62) with loop formed flow, are parked there and/or measured and moved back into the first channel.

8. A fluid flow system for manipulating micro-particles (14, 23, 33, 43, 54, 64, 73, 83) in flowing fluids, with at least one first channel (22, 32, 42, 51, 61, 72, 82) for receiving a flow of a fluid, which comprises at least one side opening (17, 24a, 24b, 35, 76a, 76b, 86) and at least one electrode arrangement (15a, 15b, 16a, 16b, 25a, 26a, 25b, 26b, 34a-e, 44a-d, 55a-c, 53a-b, 74a-b, 84a-b), which is designed to generate at least one electric field barrier passing through the flow, **characterized in that** the electrode arrangement is so formed and can be so subjected to an electric high frequency voltage that the electric field barrier for at least one micro-particle forms a guide on the upstream side of the electrode arrangement for a directed movement of the micro-particle towards the side opening and at least one second channel (21, 31, 41, 52, 62, 71, 81) is provided for reception of a further fluid flow, which has at least one side opening which forms a transition region with the side opening of the first channel.

9. A fluid flow system according to claim 8, in which at least one further electrode arrangement (15b, 16b, 25b, 26b, 34b-e, 44b, d, 55b, c, 74a, 84b) is provided in the second channel and is arranged to form at least one electric field barrier transverse or oblique to the flow direction in the first channel for movement of at least one micro-particle to a further side opening.

10. A fluid flow system according to either claim 8 or 9, in which the first and second channels are straight capillary channels, which are separated by a partition (12, 27, 45) in which the side opening(s) are formed.

11. A fluid flow system according to claim 10, in which each opening forms at least two transition regions with an electrode arrangement each.

12. A fluid flow system according to any of claims 8 to 11, in which the electrode arrangement comprises electrode sections which are fitted on the channel wall and extend from a channel side opposite the respective side opening upstream to the respective side opening.

13. A fluid flow system according to claim 12, in which the electrode sections are of strip form and project into the respective other channel.

14. A fluid flow system according to either claim 12 or 13, in which two parallel electrode sections on opposite regions of the channel wall are associated with a transition region.

15. A fluid flow system according to either claim 8 or 14, in which the second channel (62) is a loop formed branch from the first channel.

16. A fluid flow system according to either claim 8 or 14, in which a plurality of first channels and/or second channels and/or branches are provided, which are connected by transition regions, which each comprise an electrode arrangement.

17. Use of fluid flow system according to either claim 8 or 16, as
- a classifying or segregating device,
- a treatment device, and/or
- a storage or holding device.

## Revendications

1. Procédé de manipulation de microparticules (14, 23, 33, 43, 54, 64, 73, 83) dans un fluide qui parcourt sous forme de flux au moins un premier canal comportant au moins une ouverture latérale (17, 24a, 24b, 35, 76a, 76b, 86) et au moins un dispositif d'électrode (15a, 15b, 16a, 16b, 25a, 26a, 25b, 26b, 34a-e, 44a-d, 55a-c, 63a-b, 74a-b, 84a-b), conçu pour produire au moins une barrière de champ électrique, le dispositif d'électrode (15a, 15b, 16a, 16b, 25a, 26a, 25b, 26b, 34a-e, 44a-d, 55a-c, 63a-b, 74a-b, 84a-b) étant réglé par alimentation avec un courant haute fréquence électrique au moins temporairement de manière qu'il se forme une barrière de champ électrique traversant le flux, et sous l'action de laquelle une ou plusieurs microparticules (14, 23, 33, 43, 54, 64, 73, 83) ne peuvent pas passer dans le dispositif d'électrode, **caractérisé en ce que**
les microparticules (14, 23, 33, 43, 54, 64, 73, 83) du côté situé en amont du dispositif d'électrode (15a, 15b, 16a, 16b, 25a, 26a, 25b, 26b, 34a-e, 44a-d, 55a-c, 63a-b, 74a-b, 84a-b) subissent une modification de déplacement avec une direction s'écartant de la direction du flux et dirigée au bord du flux vers l'ouverture latérale (17, 24a, 24b, 35, 76a, 76b, 86), où chaque ouverture latérale (17, 24a, 24b, 35, 76a, 76b, 86) forme une zone de transition dans laquelle les microparticules (14, 23, 33, 43, 54, 64, 73, 83) se déplacent à chaque fois du premier canal (22, 32, 42, 51, 61, 72, 82) dans un second canal voisin (21, 31, 41, 52, 62, 71, 81) ou plusieurs seconds canaux voisins (52, 53) ou une dérivation du premier canal avec une formation de flux en forme de boucle, ou inversement.

2. Procédé selon la revendication 1, dans lequel chaque microparticule se déplace plus avant sous l'action de la tension haute fréquence électrique entre au moins deux microélectrodes (15a, 15b, 16a, 16b, 25a, 26a, 25b, 26b, 34a-e, 44a-d, 55a-c, 63a-b, 74a-b, 84a-b) qui produisent la barrière de champ électrique, et les forces de flux dans le fluide dans la zone de transition et sous l'action du flux de manière à chaque fois correspondante dans le canal voisin ou une dérivation de ce canal.

3. Procédé selon la revendication 2, dans lequel des champs électriques haute fréquence sont produits localement de façon limitée dans des sections de canal qui à chaque fois voisinent ou recouvrent une zone de transition.

4. Procédé selon l'une des revendications 1 à 3, dans lequel les flux dans les canaux ou dérivations sont des écoulements laminaires.

5. Procédé selon l'une des revendications 1 à 4, dans lequel les microparticules (54) sont triées par un premier canal (51) dans un grand nombre de seconds canaux (52, 53) selon leur spécificité particulaire, du fait que des barrières de champ électrique sont formées localement pour chaque microparticule de façon spécifique correspondant à un modèle temporel prédéterminé, afin que se produise un mouvement dans un canal prédéterminé du groupe des seconds canaux.

6. Procédé selon l'une des revendications 1 à 4, dans lequel le fluide dans le premier canal (22) est un liquide support inerte et le fluide dans le second canal (21) est un liquide de traitement et les barrières de champ électrique sont formées de manière prédéterminée, de façon que les microparticules (23), en correspondance avec un modèle temporel prédéterminé passent du liquide support au liquide de traitement, y soient traitées et retournent à nouveau dans le liquide support.

7. Procédé selon l'une des revendications 1 à 4, dans lequel les microparticules (64), de façon correspondante à un modèle temporel prédéterminé, se déplacent du premier canal (61) dans une dérivation en forme de boucle ou un réservoir (62) avec flux en forme de boucle, y sont parquées et/ou mesurées et renvoyées à nouveau dans le premier canal.

8. Système de flux de fluide pour la manipulation de microparticules (14, 23, 33, 43, 54, 64, 73, 83) dans des fluides en écoulement avec au moins un premier canal (22, 32, 42, 51, 61, 72, 82) pour capter un flux de fluide, qui présente au moins une ouverture latérale (17, 24a, 24b, 35, 76a, 76b, 86) et au moins un dispositif d'électrode (15a, 15b, 16a, 16b, 25a, 26a, 25b, 26b, 34a-e, 44a-d, 55a-c, 63a-b, 74a-b, 84a-b) qui est formé pour produire au moins une barrière de champ électrique traversant le flux,
**caractérisé en ce que**
le dispositif d'électrode est formé de manière, et alimenté avec une tension haute fréquence électrique telle que pour au moins une microparticule la barrière de champ électrique du côté disposé en amont du dispositif d'électrode forme guide pour un déplacement orienté de la microparticule en direction de l'ouverture latérale, et **en ce qu'**est prévu au moins un second canal (21, 31, 41, 52, 62, 71, 81) pour capter un autre flux de fluide, qui présente au moins une ouverture latérale formant avec l'ouverture latérale du premier canal une zone de transition.

9. Système de flux de fluide selon la revendication 8, dans lequel dans le second canal est prévu au moins un autre dispositif d'électrode (15b, 16b, 25b, 26b, 34b-e, 44b, d, 55b, c, 74a, 84b) qui est monté en vue de formert au moins une barrière de champ électrique perpendiculaire ou oblique par rapport à la direction du flux dans le premier canal en vue du déplacement d'au moins une microparticule vers une autre ouverture latérale.

10. Système de flux de fluide selon l'une des revendications 8 ou 9, dans lequel les premier et second canaux sont des canaux capillaires droits qui sont séparés par une paroi de séparation (12, 27, 45) dans laquelle est ou sont formée(s) la ou les ouverture(s) latérale(s).

11. Système de flux de fluide selon la revendication 10, dans lequel chaque ouverture forme au moins deux zones de transition avec à chaque fois un dispositif d'électrode.

12. Système de flux de fluide selon l'une des revendications 8 à 11, dans lequel le dispositif d'électrode comprend des sections d'électrode qui sont appliquées à la paroi du canal et s'étendent d'un côté du canal opposé à l'ouverture latérale considérée en direction de l'ouverture latérale en question.

13. Système de flux de fluide selon la revendication 12, dans lequel les sections d'électrode sont en forme de ruban et dépassent dans l'autre canal considéré.

14. Système de flux de fluide selon l'une des revendications 12 ou 13, dans lequel à une zone de transition sont affectées à chaque fois deux sections d'électrode parallèles à des endroits opposés de la paroi du canal.

15. Système de flux de fluide selon l'une des revendications 8 ou 14, dans lequel le second canal (62) est une dérivation en forme de boucle du premier canal.

16. Système de flux de fluide selon l'une des revendications 8 ou 14, dans lequel est prévu un grand nombre de premiers canaux et/ou de seconds canaux et/ou de dérivations, qui sont reliés par des zones de transition présentant à chaque fois un dispositif d'électrode.

17. Utilisation d'un système de flux de fluide selon l'une des revendications 8 ou 16, comme
- dispositif de tri ou de séparation ;
- dispositif de traitement, et/ou
- dispositif de conservation et de fixation.
